# EUROPÄISCHE PATENTSCHRIFT

(11) **EP 2 595 671 B1**
(45) Veröffentlichungstag und Bekanntmachung des Hinweises auf die Patenterteilung: **26.02.2020**
(21) Anmeldenummer: 11736006.5
(22) Anmeldetag: 22.07.2011
(51) Int. Cl.: A61M 1/36, B08B 9/032

(54) **SPÜLLEITUNG, MEDIZINTECHNISCHE FUNKTIONSEINRICHTUNG, MEDIZINTECHNISCHE BEHANDLUNGSVORRICHTUNG SOWIE VERFAHREN**
RINSE CONDUIT, MEDICO-TECHNICAL FUNCTIONAL DEVICE, MEDICO-TECHNICAL TREATMENT DEVICE AND METHOD
CONDUITE DE SPÜLLEITUNG, DISPOSITIF FONCTIONNEL MÉDICAL, DISPOSITIF DE TRAITEMENT MÉDICAL ET PROCÉCÉ

(30) Priorität: 23.07.2010 DE 102010032182
(43) Veröffentlichungstag der Anmeldung: 29.05.2013
(73) Patentinhaber: Fresenius Medical Care Deutschland GmbH, 61352 Bad Homburg (DE)
(72) Erfinder: HAECKER, Juergen, 61267 Neu-Anspach (DE); GRONAU, Soeren, 64569 Bad Nauheim (DE)
(74) Vertreter: Bobbert & Partner Patentanwälte PartmbB
(86) Internationale Anmeldenummer: PCT/EP2011/003682
(87) Internationale Veröffentlichungsnummer: WO 2012/010320

(56) Entgegenhaltungen:
- EP-A1- 1 454 643
- EP-A2- 0 560 368
- EP-A2- 1 057 493
- WO-A1-99/20376
- WO-A1-02/062454
- WO-A1-2008/028579
- DE-A1-102006 022 122
- DE-C1- 19 824 015
- US-A1- 2008 146 996
- US-A1- 2010 116 740

## Beschreibung

Die vorliegende Erfindung betrifft eine Spülleitung gemäß Anspruch 1. Sie betrifft ferner eine medizintechnische Funktionseinrichtung gemäß Anspruch 4, sowie ein Verfahren gemäß Anspruch 7.

Das Spülen medizintechnischer Funktionseinrichtungen, z. B. vor und/oder nach deren Einsatz im Rahmen einer medizinischen Behandlung, ist aus der Praxis bekannt.

EP 1 454 643 A1 und WO 02/062454 A1 offenbaren Blutbehandlungsvorrichtungen sowie Verfahren zum Betreiben solcher Vorrichtungen. Insbesondere offenbaren WO 2008/028579 A1, WO 99/20376 A1 und DE 10 2006 022122 A1 verschiedene Verfahren zum Entleeren und/oder zum Primen und/oder zur Füllung und Spülung solcher Vorrichtungen. US 2008/146996 A1 offenbart schließlich eine Steuereinrichtung zur Kontrolle eines Pumpsystems, welches in einem solchen Verfahren verwendet wird.

Eine Aufgabe der vorliegenden Erfindung ist, eine zu diesem Zweck geeignete Spülleitung anzugeben. Ferner soll ein geeignetes Verfahren zum Spülen und/oder Primen einer medizintechnischen Funktionseinrichtung angegeben werden.

Diese Aufgabe wird durch eine Spülleitung mit den Merkmalen des Anspruchs 1 gelöst. Sie wird ferner durch eine medizintechnische Funktionseinrichtung mit den Merkmalen des Anspruchs 4 sowie ein Verfahren mit den Merkmalen des Anspruchs 7 gelöst.

Alle mit der erfindungsgemäßen Spülleitung erzielbaren Vorteile lassen sich ungeschmälert auch mit der medizintechnischen Funktionseinrichtung und/oder der medizintechnischen Behandlungsvorrichtung und/oder dem Verfahren und/oder der Steuereinrichtung erzielen.

Erfindungsgemäß wird eine Spülleitung vorgeschlagen, die zum Abführen eines Spülfluids aus einer mittels des Spülfluids gespülten medizintechnischen Funktionseinrichtung heraus geeignet und vorgesehen ist.

Einerseits oder an einem ihrer Enden oder Fluidverbindungsabschnitte ist die erfindungsgemäße Spülleitung verbunden oder steht in Fluidverbindung mit einem Abschnitt der medizintechnischen Funktionseinrichtung, oder die erfindungsgemäße Spülleitung ist zu ihrer Verbindung mit dem Abschnitt vorgesehen.

Andererseits oder an einem ihrer Enden oder Fluidverbindungsabschnitte ist die erfindungsgemäße Spülleitung verbunden mit - oder zu ihrer Verbindung vorgesehen mit - einer Fluidzuführeinrichtung zum Zuführen oder Einleiten eines medizinischen Fluids in die medizintechnische Funktionseinrichtung hinein.

Die erfindungsgemäße medizintechnische Funktionseinrichtung (im Folgenden auch kurz: Funktionseinrichtung) ist vorgesehen zur Verbindung mit oder verbunden mit wenigstens einer Spülleitung gemäß der vorliegenden Erfindung.

Die hierin offenbarte medizintechnische Behandlungsvorrichtung (im Folgenden auch kurz: Behandlungsvorrichtung) ist vorgesehen zur Verbindung mit oder verbunden mit wenigstens einer Spülleitung gemäß der vorliegenden Erfindung und/oder mit einer medizintechnischen Funktionseinrichtung gemäß der vorliegenden Erfindung.

Das erfindungsgemäße Verfahren dient dem Spülen und/oder Primen einer medizintechnischen Funktionseinrichtung bzw. Funktionseinrichtung und umfasst das Verwenden einer Spülleitung gemäß der vorliegenden Erfindung oder einer erfindungsgemäßen medizinischen Funktionseinrichtung.

Die hierin offenbarte Steuereinrichtung ist vorgesehen und konfiguriert zur automatisierten Durchführung wenigstens eines der Schritte eines Spül- und/oder Primingverfahrens, insbesondere eines Spül- und/oder Primingverfahrens gemäß der vorliegenden Erfindung.

Die Steuereinrichtung ist insbesondere vorgesehen zur Verwendung mit einer medizintechnischen Behandlungsvorrichtung.

Vorteilhafte Weiterbildungen der vorliegenden Erfindung sind jeweils Gegenstand der Unteransprüche.

Erfindungsgemäße Ausführungsformen können einige oder alle der folgenden Merkmale in beliebiger Kombination aufweisen.

Der Begriff "Abführen", wie er hierin verwendet wird, bezeichnet in bestimmten Ausführungsformen der vorliegenden Erfindung ein Austragen und/oder Ab- oder Ausleiten eines Spülfluids aus der Funktionseinrichtung heraus, vorzugsweise über die Fluidzuführeinrichtung, die in diesem Fall auch zum Zuführen, nicht nur zum Abführen ausgestaltet und vorgesehen ist, weiter vorzugsweise in eine Behandlungsvorrichtung hinein.

Unter einem "Spülfluid" ist in bestimmten Ausführungsformen der vorliegenden Erfindung ein Fluid zu verstehen, welches zum Spülen und/oder Primen einer Funktionseinrichtung geeignet und vorgesehen ist.

Nicht einschränkende Beispiele schließen Substituatflüssigkeit, Sterilisationsfluide und dergleichen, Gase, wie Sterilluft, sowie Kombinationen oder Mischungen derselben, ein.

Eine Verbindung der Spülleitung mit einem Abschnitt der Funktionseinrichtung meint in bestimmten Ausführungsformen der vorliegenden Erfindung, dass die Spülleitung zur Fluidverbindung mit einem fluidführenden Inneren dieses Abschnitts der Funktionseinrichtung vorgesehen ist oder mit diesem in Fluidverbindung steht. In solchen Ausführungsformen ist ein Fluidaustausch zwischen einem Inneren der Spülleitung und dem Inneren des Abschnitts der Funktionseinrichtung vorgesehen.

Der Abschnitt der medizintechnischen Funktionseinrichtung bzw. Funktionseinrichtung ist in manchen erfindungsgemäßen Ausführungsformen ein zumindest in bestimmten Verfahren, bei denen ein Patient unter bestimmungsgemäßer Verwendung der Funktionseinrichtung behandelt wird, ein Fluid (z. B. Blut oder Spülflüssigkeit) führender Abschnitt.

Eine Verbindung der Spülleitung mit einer Fluidzuführeinrichtung bedeutet in bestimmten Ausführungsformen der vorliegenden Erfindung, dass die Spülleitung zur Fluidverbindung mit einem fluidführenden Inneren der Fluidzuführeinrichtung vorgesehen ist oder mit dieser in Fluidverbindung steht.

Der Begriff "Fluidzuführeinrichtung", wie er hierin verwendet wird, bezeichnet eine Einrichtung, welche zum Zuführen und/oder Abführen wenigstens eines medizinischen Fluids in die Funktionseinrichtung hinein oder aus der Funktionseinrichtung heraus geeignet und/oder vorgesehen ist.

Ein "medizinisches Fluid" bezeichnet in bestimmten Ausführungsformen der vorliegenden Erfindung ein Behandlungsfluid, das während der Behandlung des Patienten zum Einsatz kommt oder kommen kann. Nicht einschränkende Beispiele hierfür schließen allgemein Flüssigkeiten, wie Blut, Dialysat, Substituatflüssigkeit, Medikamentenlösungen, Priming- und/oder Spül- und/oder Sterilisationsfluide und dergleichen, sowie Kombinationen oder Mischungen derselben ein.

Das medizinische Fluid und das Spülfluid können in bestimmten Ausführungsformen der vorliegenden Erfindung gleicher Art und/oder identisch sein. Dies muss aber nicht der Fall sein.

In manchen erfindungsgemäßen Ausführungsformen ist das Spülfluid das zuvor als medizinisches Fluid eingeleitete Fluid. In bestimmten erfindungsgemäßen Ausführungsformen ist das Spülfluid ein Substituat, welches z. B. bei bestimmten Dialyseverfahren zum Einsatz kommt. In einigen erfindungsgemäßen Ausführungsformen werden sowohl das medizinische Fluid als auch das Spülfluid der Funktionseinrichtung über die Fluidzuführeinrichtung zugeführt. In manchen erfindungsgemäßen Ausführungsformen werden sowohl das medizinische Fluid als auch das Spülfluid aus der Funktionseinrichtung über die Fluidzuführeinrichtung abgeführt. In bestimmten erfindungsgemäßen Ausführungsformen werden sowohl das medizinische Fluid als auch das Spülfluid online in der zur Behandlung des Patienten eingesetzten Behandlungsvorrichtung erzeugt.

Die Fluidzuführeinrichtung ist in bestimmten Ausführungsformen der vorliegenden Erfindung ein Verbindungsabschnitt oder Teil eines Verbindungsabschnitts, welcher zum Herstellen einer Fluidverbindung, vorzugsweise einer unmittelbaren Fluidverbindung, zwischen der Funktionseinrichtung und der medizintechnischen Behandlungsvorrichtung bzw. Behandlungsvorrichtung vorgesehen ist.

Der Verbindungsabschnitt oder die Fluidzuführeinrichtung ist in bestimmten Ausführungsformen der vorliegenden Erfindung zumindest auch dazu vorgesehen, das medizinische Fluid von der medizintechnischen Behandlungsvorrichtung in die medizintechnische Funktionseinrichtung einzuleiten oder einzutragen oder von der medizintechnischen Behandlungsvorrichtung zur medizintechnischen Funktionseinrichtung zu überführen, zu übertragen und dergleichen.

In bestimmten Ausführungsformen der vorliegenden Erfindung ist die Fluidzuführeinrichtung ein Port zum Zuführen von Substituatflüssigkeit in die medizinische Funktionseinrichtung hinein.

In manchen Ausführungsformen erfolgt das Zuführen von Substituatflüssigkeit im Rahmen einer extrakorporalen Behandlung als Prä- und/oder Postdilution.
Die Fluidzuführeinrichtung ist gemäß der vorliegenden Erfindung ein zweilumiger Verbindungsabschnitt oder weist einen solchen auf.

Ein solcher zweilumiger Verbindungsabschnitt ist erfindungsgemäß zum gleichzeitigen Führen von wenigstens zwei Fluiden, bei welchen es sich um verschiedene Fluide handelt, geeignet und/oder vorgesehen.

Der zweilumige Verbindungsabschnitt weist in bestimmten Ausführungsformen ein erstes Lumen auf, welches zur Aufnahme und/oder Leitung des medizinischen Fluids vorgesehen ist. Er weist ferner ein zweites Lumen auf, welches zur Aufnahme und/oder Leitung des Spülfluids vorgesehen ist.

In solchen Ausführungsformen steht insbesondere das erste Lumen des zweilumigen Verbindungabschnitts mit einer Fluidleitung der Funktionseinrichtung für das medizinische Fluid in Fluidverbindung; das zweite Lumen des zweilumigen Verbindungabschnitts steht mit der Spülleitung für das Spülfluid in Fluidverbindung oder umgekehrt.

Der Begriff "gleichzeitiges Führen" von wenigstens zwei Fluiden bezeichnet in bestimmten Ausführungsformen der vorliegenden Erfindung, das zeitgleiche, d. h. zum selben Zeitpunkt, im selben Zeitintervall und dergleichen erfolgende Aufnehmen und/oder Führen oder Leiten der Fluide, z. B. des medizinischen Fluids und des Spülfluids.

Dabei können das Spülfluid und das medizinische Fluid in Gegenrichtung zueinander strömen. Sie können jedoch auch in einer nicht entgegengesetzten Richtung zueinander strömen.

In bestimmten Ausführungsformen der vorliegenden Erfindung ist die Funktionseinrichtung ein extrakorporaler Blutkreislauf oder weist einen solchen auf.

In manchen Ausführungsformen der vorliegenden Erfindung ist die Funktionseinrichtung eine Blutkassette oder weist eine solche auf.

In bestimmten Ausführungsformen der vorliegenden Erfindung ist der Abschnitt der als extrakorporaler Blutkreislauf ausgestalteten Funktionseinrichtung ein Abschnitt eines arteriellen Leitungsabschnitts des extrakorporalen Blutkreislaufs.

In bestimmten Ausführungsformen der vorliegenden Erfindung ist der Abschnitt der als extrakorporaler Blutkreislauf ausgestalteten Funktionseinrichtung zwischen einer Blutpumpe oder einem Eingriffsabschnitt des extrakorporalen Blutkreislaufs für eine Blutpumpe und einer extrakorporalen Blutbehandlungseinrichtung wie z. B. einem Blutfilter oder einem Dialysator angeordnet.

Eine "Blutpumpe", wie hierin verwendet, bezeichnet beispielsweise eine Verdrängerpumpe, wie eine Schlauchpumpe oder peristaltische Pumpe, oder jede andere Pumpe, welche dazu vorgesehen und/oder ausgelegt ist, während einer extrakorporalen Blutbehandlung Blut (z. B. mittels Verdrängung) aus einem ersten Abschnitt des extrakorporalen Blutkreislaufs in einen zweiten Abschnitt des extrakorporalen Blutkreislaufs zu fördern.

Der Begriff "Eingriffsabschnitt für eine Blutpumpe", wie er hierin verwendet wird, bezeichnet in bestimmten Ausführungsformen der vorliegenden Erfindung einen Abschnitt des extrakorporalen Blutkreislaufs, an welchem die Blutpumpe bei ihrem bestimmungsgemäßem Einsatz zum Verdrängen oder Fördern des Bluts mit diesem in Beziehung tritt oder mit dem extrakorporalen Blutkreislauf zu diesem Zweck verbunden wird.

Beispiele für eine extrakorporale Blutbehandlungseinrichtung umfassen einen Blutfilter zum Reinigen von Patientenblut im Rahmen einer Hämodialyse- und/oder Hämofiltrationsbehandlung und/oder Hämodiafiltrationsbehandlung und dergleichen, sind jedoch nicht hierauf beschränkt. Die extrakorporale Blutbehandlungseinrichtung kann als Einwegprodukt oder Disposable-Einrichtung ausgestaltet sein.

In bestimmten Ausführungsformen der vorliegenden Erfindung ist der Abschnitt des extrakorporalen Blutkreislaufs zwischen der Blutpumpe oder dem Eingriffsabschnitt des extrakorporalen Blutkreislaufs für die Blutpumpe des extrakorporalen Blutkreislaufs und einem Prädilutionszugang des extrakorporalen Blutkreislaufs angeordnet.

In manchen Ausführungsformen der vorliegenden Erfindung ist die erfindungsgemäße Spülleitung ein zusätzliches Element der Funktionseinrichtung. "Zusätzlich" bedeutet hier, dass die Funktionseinrichtung auch verwendet werden kann, falls die erfindungsgemäße Spülleitung nicht zum Einsatz kommt. Insbesondere können ohne Verwenden der erfindungsgemäßen Spülleitung bei entsprechender Ausgestaltung der Funktionseinrichtung alle Verfahren und Funktionen ausgeführt werden, wie sie in der DE 10 2009 018 664.6 der Anmelderin der vorliegenden Anmeldung mit dem Titel *"Externe Funktionseinrichtung, Blutbehandlungsvorrichtung zum Aufnehmen einer erfindungsgemäßen externen Funktionseinrichtung, sowie Verfahren",* die am 23. April 2009 beim Deutschen Patent- und Markenamt eingereicht wurde, beschriebenen Ausführungsformen, sowie der Anmeldung DE 10 2009 024 468.9 mit demselben Titel der Anmelderin der vorliegenden Anmeldung, die am 10. Juni 2009 beim Deutschen Patent- und Markenamt eingereicht wurde. Allein die besonderen Funktionen, die mittels der erfindungsgemäßen Spülleitung erfindungsgemäß ermöglicht werden sollen, wären mittels der Funktionseinrichtung, welche keine erfindungsgemäße Spülleitung aufweist, nicht ausführbar. In solchen Ausführungsformen kann die Spülleitung nach ihrem Herstellen mit der Funktionseinrichtung und der Fluidzuführeinrichtung verbunden werden. Sie kann, ohne hierauf beschränkt zu sein, auf- und/oder angesteckt, auf- und/oder angeschraubt, angeclipt oder in sonstiger Weise lösbar befestigt oder unlösbar befestigt, wie beispielsweise angeschweißt, angeklebt und dergleichen, angebracht werden. Sie kann alternativ allerdings auch ab Werk, integral oder einstückig mit der erfindungsgemäßen Spülleitung verbunden oder hergestellt sein. Auch in diesem Fall kann die erfindungsgemäße Spülleitung als zusätzliches Element der Funktionseinrichtung bezeichnet werden.

In bestimmten Ausführungsformen der vorliegenden Erfindung ist die Spülleitung als Strömungskanal in die Funktionseinrichtung integriert bzw. integral mit dieser hergestellt. In solchen Ausführungsformen kann die Spülleitung - wie vorstehend beschrieben - nach ihrer Herstellung mit der Fluidzuführeinrichtung verbunden werden.

In bestimmten Ausführungsformen der vorliegenden Erfindung ist die Spülleitung mittels eines automatischen oder elektiv betätigbaren Spülventils mit dem Abschnitt der Funktionseinrichtung verbunden oder zur Verbindung vorgesehen.

In manchen erfindungsgemäßen Ausführungsformen ist das Spülventil Bestandteil der Spülleitung, in anderen Ausführungsformen ist es Bestandteil der Funktionseinrichtung.

In bestimmten erfindungsgemäßen Ausführungsformen ist die Spülleitung mittels des Spülventils verschließbar und/oder kann hiermit geöffnet werden.

In manchen erfindungsgemäßen Ausführungsformen ist das Spülventil als ein Phantomventil ausgestaltet.

Der Begriff "Phantomventil", wie er hierin verwendet wird, bezeichnet ein Element mit einer mittels Aktor erreichbaren Aktor-Fläche (hier beispielsweise eine Aktor-Membran), welche die Funktion eines Ventils übernehmen kann.

Die Aktor-Membran ist unter Aufbringung einer Kraft hierauf, z. B. einer Druckkraft, in eine Richtung bewegbar, ausdehnbar bzw. wölbbar oder dergleichen. Durch das Bewegen oder Ausdehnen der Aktor-Membran kann sich diese an ein Element, wie eine Abdichtungseinrichtung, etwa einen Steg, anlegen oder von diesem entfernen. Die Aktor-Membran kann somit beispielsweise eine Abdichtung bewirken bzw. verstärken oder beenden bzw. verringern.

Wenn die Kraft wieder von der Aktor-Membran genommen wird, kann diese in beispielsweise eine Grundposition, z. B. einen nicht gewölbten Zustand, zurückkehren.

Ein Phantomventil kann mit oder aus einem Stegabschnitt eines Kanals an einem Hartteil der Blutkassette und einem dem Stegabschnitt an- oder gegenüberliegenden Abschnitt einer Folie hiervon ausgestaltet sein oder werden.

Phantomventile können durch Aktoren der Blutbehandlungsvorrichtung betätigt werden.

Zum Schließen eines Phantomventils kann der Abschnitt einer Folie (welche in bestimmten Ausführungsformen zum Abdecken des Hartteils der Blutkassette eingesetzt wird) auf den Stegabschnitt gedrückt werden. Zum Öffnen des Phantomventils kann der Abschnitt der Folie wieder vom Stegabschnitt abgehoben werden.

Weitere Beispiele und/oder Ausführungsformen für Phantomventile können der Patentanmeldung DE 10 2009 012 632.5 mit dem Titel *"Abdichtungseinrichtung zum Abdichten eines Volumens einer medizinischen Behandlungsanordnung gegen ein weiteres Volumen sowie Anordnung und Verfahren",* die am 10. März 2009 von der vorliegenden Anmelderin beim DPMA eingereicht wurde, entnommen werden.

In bestimmten Ausführungsformen weist die Funktionseinrichtung wenigstens eine Ankoppeleinrichtung als Port oder Fluidzuführeinrichtung auf, welcher einen Innenrohrabschnitt mit einem ersten Lumen und einen Außenrohrabschnitt mit einem zweiten Lumen aufweist, wobei der Außenrohrabschnitt wenigstens um einen Bereich des Innenrohrabschnitts derart angeordnet ist, dass ein Raum innerhalb des zweiten Lumens zwischen dem Äußeren des Innenrohrabschnitts und dem Außenrohrabschnitt ausgestaltet ist, wobei das erste Lumen des Innenrohrabschnitts vorgesehen ist zur Aufnahme und/oder Führung des medizinischen Fluids und der Raum des Außenrohrabschnitts zur Aufnahme und/oder Führung wenigstens des Spülfluids vorgesehen ist oder umgekehrt.

Geeignete Beispiele für einen solchen Port schließen die in der am 23. Juli 2010 beim Deutschen Patent- und Markenamt unter dem amtlichen Aktenzeichen DE 10 2010 032 181.8 eingereichten Anmeldung der vorliegenden Anmelderin mit dem Titel *"Ankoppeleinrichtung, Konnektor, medizintechnische Funktionseinrichtung, medizintechnische Behandlungsvorrichtung sowie Verfahren"* offenbarten Ausführungen ein.

In bestimmten Ausführungsformen der vorliegenden Erfindung ist die Funktionseinrichtung als extrakorporaler Blutschlauchsatz ausgestaltet oder weist einen solchen auf.

In bestimmten Ausführungsformen der vorliegenden Erfindung ist die Funktionseinrichtung als Blutkassette ausgestaltet, oder Teil einer solchen, oder weist eine solche auf. Eine solche Blutkassette kann zum Beispiel als Gussteil oder Spritzgussteil ausgestaltet sein. Sie kann unabhängig hiervon als Einweg-Blutkassette oder Disposable-Blutkassette ausgestaltet sein.

Beispiele für geeignete Blutkassetten schließen die in der Anmeldung DE 10 2009 018 664.6 der Anmelderin der vorliegenden Anmeldung mit dem Titel *"Externe Funktionseinrichtung, Blutbehandlungsvorrichtung zum Aufnehmen einer erfindungsgemäßen externen Funktionseinrichtung, sowie Verfahren",* die am 23. April 2009 beim Deutschen Patent- und Markenamt eingereicht wurde, beschriebenen Ausführungsformen, sowie die in der Anmeldung DE 10 2009 024 468.9 von der Anmelderin der vorliegenden Anmeldung mit dem Titel *"Externe Funktionseinrichtung, Blutbehandlungsvorrichtung zum Aufnehmen einer erfindungsgemäßen externen Funktionseinrichtung, sowie Verfahren",* die am 10. Juni 2009 beim Deutschen Patent- und Markenamt eingereicht wurde, beschriebenen Ausführungsformen ein, ohne auf diese beschränkt zu sein.

Die offenbarte Behandlungsvorrichtung ist in bestimmten Ausführungsformen als extrakorporale Behandlungsvorrichtung, insbesondere als extrakorporale Blutbehandlungsvorrichtung, etwa als Dialysiervorrichtung - beispielsweise zur Verwendung im Rahmen einer Single-Needle- oder Double-Needle-Dialysebehandlung - insbesondere als Hämodialysiervorrichtung, Hämofiltrationsvorrichtung, Hämodiafiltrationsvorrichtung, oder als Vorrichtung für die Adsorption, Leberersatztherapie, Apherese, Transfusion, usw. ausgestaltet.

In bestimmten Ausführungsformen des erfindungsgemäßen Verfahrens ist vorgesehen, in der gespülten medizintechnischen Funktionseinrichtung vorhandenes Spülfluid vollständig oder wenigstens teilweise durch die Spülleitung in einen Port oder Maschinenanschluss zum Einbringen von Substituatflüssigkeit aus der medizintechnischen Behandlungsvorrichtung in die medizintechnische Funktionseinrichtung auszutragen bzw. abzuleiten.

In bestimmten Ausführungsformen des erfindungsgemäßen Verfahrens wird in einem ersten Schritt Substituatflüssigkeit in die Funktionseinrichtung eingebracht.

Die Substituatflüssigkeit wird in einem Inneren der Funktionseinrichtung gefördert, um die Funktionseinrichtung zu spülen und/oder zu primen.

In bestimmten Ausführungsformen des erfindungsgemäßen Verfahrens erfolgt das Spülen und/oder Primen mit einer bestimmten Spülmenge an Substituatflüssigkeit.

In manchen erfindungsgemäßen Ausführungsformen wird die Spülleitung nach Beenden des Spülvorgangs verschlossen. Eine entsprechende Einrichtung kann an einem oder beiden Enden der Spülleitung vorgesehen sein.

Alle, einige oder manche Schritte eines erfindungsgemäßen Verfahrens, wie es beispielhaft und nicht einschränkend anhand der beigefügten Zeichnung beschrieben ist, können automatisch und/oder automatisiert durchgeführt werden.

Ferner können auch ein digitales Speichermedium, ein Computer-Programm-Produkt sowie ein Computer-Programm bei der Lösung der Aufgabe unterstützend eingesetzt werden.

Ein digitales Speichermedium, insbesondere in Form einer Diskette, eines RAM, ROM, einer CD oder DVD, mit elektrisch auslesbaren Steuersignalen kann derart mit einem programmierbaren Computersystem zusammenwirken, dass die maschinellen Schritte eines erfindungsgemäßen Verfahrens veranlasst werden.

Dabei können alle, einige oder manche der maschinell durchgeführten Schritte des erfindungsgemäßen Verfahrens veranlasst werden.

Ein Computer-Programm-Produkt weist einen auf einem maschinenlesbaren Träger gespeicherten Programmcode zur Veranlassung der maschinellen Schritte des erfindungsgemäßen Verfahrens, wenn das Computer-Programm-Produkt auf einem Rechner abläuft, auf.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, ein RAM, ROM, eine CD, DVD, ein USB-Stick, eine Flashcard, eine SD-Karte und dergleichen sein.

Ein Computer-Programm weist einen Programmcode auf zur Veranlassung der maschinellen Schritte eines erfindungsgemäßen Verfahrens, wenn das Computer-Programm auf einem Computer abläuft.

Auch für das Computer-Programm-Produkt und das Computer-Programm gilt, dass bei seinem Ausführen alle, einige oder manche der maschinell durchgeführten Schritte des erfindungsgemäßen Verfahrens veranlasst werden.

Bestimmte erfindungsgemäße Ausführungsformen weisen einen oder mehrere der im Folgenden genannten Vorteile auf.

Die vorliegende Erfindung stellt eine Spülleitung bereit, welche in manchen erfindungsgemäßen Ausführungsformen vorteilhaft auf einfache Weise dazu beitragen kann, den konstruktiven Aufwand beim Herstellen einer Anordnung zur Behandlung eines Patienten zu senken. Dies trägt wiederum in vorteilhafter Weise dazu bei, den Kostenaufwand zu reduzieren.

Da die erfindungsgemäße Spülleitung ein separates Bauteil der Funktionseinrichtung bildet, kann ein extrakorporaler Blutkreislauf in bestimmten Ausführungsformen der vorliegenden Erfindung mit vorkonnektierten Leitungsabschnitten (arterielle und venöse Patientenleitung) ausgeliefert werden, so dass vorteilhaft auf ansonsten zum Schutz der Patientenleitungen erforderliche Abdeckkappen verzichtet werden kann. Letzteres kann zu Kosteneinsparung und zur Verringerung eines Kontaminationsrisikos beitragen.

Da die erfindungsgemäße Spülleitung in einfacher Weise mit einem Port der Funktionseinrichtung verbindbar ist, kann in bestimmten Ausführungsformen der vorliegenden Erfindung vorteilhaft auf einen maschinenseitigen Rinseport sowie mindestens ein Hydraulikventil verzichtet werden. Dies kann vorteilhaft ohne Mehraufwand an einem bestehenden Port zum Zuführen von Substituatflüssigkeit, insbesondere einem Port, wie er in der am 23. Juli 2010 beim Deutschen Patent- und Markenamt eingereichten Anmeldung DE 10 2010 032 181.8 der vorliegenden Anmelderin mit dem Titel *"Ankoppeleinrichtung, Konnektor, medizintechnische Funktionseinrichtung, medizintechnische Behandlungsvorrichtung sowie Verfahren"* offenbart ist, erreicht werden.

Da es in bestimmten Ausführungsformen der vorliegenden Erfindung vorteilhaft möglich ist, ein einfaches Übergangsstück zur Verbindung mit dem Rinseport der Maschine zu verwenden, kann es vorteilhaft möglich sein, auf Disposable-Bauteile, wie beispielsweise einen Rinseportadapter mit wenigstens drei Abdeckkappen, wie er im Stand der Technik bekannt ist, zu verzichten.

Auf diese Weise kann es vorteilhaft möglich sein, Kosten für die Herstellung und/oder Verwendung der erfindungsgemäßen Funktionseinrichtung, insbesondere in Form einer Disposable-Blutkassette, zu verringern.

In bestimmten Ausführungsformen stellt die vorliegende Erfindung vorteilhaft Usability- und/oder Hygienevorteile bereit, da auf manuelle fluidische Konnektionen vorteilhaft verzichtet werden kann. Dies kann wiederum zum Einsparen von Arbeitszeit und -mühe und zum Verbessern der Ergonomie beitragen. Ferner kann es einen Beitrag zum Erhöhen einer Sicherheit gegen Kontamination leisten.

Im Folgenden wird die vorliegende Erfindung unter Bezugnahme auf die beigefügten Figuren beschrieben. In der Zeichnung bezeichnen identische Bezugszeichen gleiche oder identische Elemente. Es gilt:
- Fig. 1: zeigt eine Vorderansicht einer medizintechnischen Funktionseinrichtung der vorliegenden Erfindung; und
- Fig. 2: zeigt schematisch vereinfacht eine medizintechnische Funktionseinrichtung zum Durchführen eines erfindungsgemäßen Spül- und/oder Primingverfahrens.

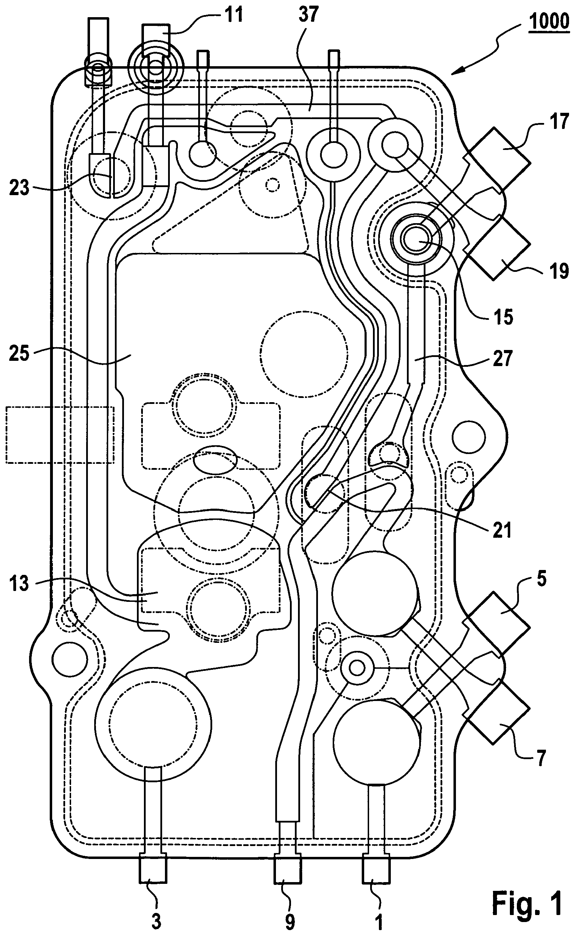

**Fig. 1** zeigt eine Vorderansicht einer erfindungsgemäßen medizintechnischen Funktionseinrichtung 1000.

Die Funktionseinrichtung 1000 ist als Blutkassette (im Folgenden kurz: Kassette) ausgestaltet.

Die Kassette 1000 weist einen arteriellen Patientenanschluss 1 sowie einen venösen Patientenanschluss 3 auf.

Die Kassette 1000 weist einen Konnektor 5 für den Blutaustritt aus der Blutkassette 1000 sowie einen Konnektor 7 für den Bluteintritt in die Kassette 1000 auf.

Die beiden Konnektoren 5 und 7 sind mit einem Pumpschlauchsegment oder -set einer Blutpumpe (in Fig. 1 nicht gezeigt) verbindbar oder verbunden.

Die Kassette 1000 weist eine arterielle Filterleitung 9 sowie eine venöse Filterleitung 11 auf.

Das Innere der Kassette 1000 weist eine venöse Blutkammer 13 auf.

Die Kassette 1000 weist eine Zugabestelle 15 für Substituatflüssigkeit in die Kassette 1000 hinein auf. Bei der Substituatflüssigkeit kann es sich um online von der Behandlungsvorrichtung hergestellte Substituatflüssigkeit handeln.

Für weitere Details zu Elementen oder Bestandteilen der in Fig. 1 gezeigten Kassette wird auf die oben genannten Anmeldungen DE 10 2009 018 664.6 und DE 10 2009 024 468.9 der Anmelderin der vorliegenden Anmeldung, insbesondere in den Fig. 17 bis 24 dort, verwiesen.

Die Zugabestelle 15 für Substituatflüssigkeit weist einen Substituat-Port (in Fig. 1 nicht gezeigt) auf.

Der Substituat-Port kann ein Port sein, wie er in der am 23. Juli 2010 beim Deutschen Patent- und Markenamt eingereichten Anmeldung DE 10 2010 032 181.8 mit dem Titel *"Ankoppeleinrichtung, Konnektor, medizintechnische Funktionseinrichtung, medizintechnische Behandlungsvorrichtung sowie Verfahren"* beschrieben ist.

Die Kassette 1000 weist einen Konnektor 17 für einen Substituataustritt aus der Kassette 1000 sowie einen Konnektor 19 für einen Sustituateintritt in die Kassette 1000 auf, wobei die Konnektoren 17 und 19 zum Zwecke des Förderns der Substituatflüssigkeit in einem Inneren der Kassette 1000 mit einem Pumpschlauchsegment oder -set einer Substituatpumpe (in Fig. 1 nicht gezeigt) verbindbar sind.

Die Kassette 1000 weist eine Substituatleitung 37, einen Prädilutionszugang 21 und einen Postdilutionszugang 23 auf.

Die - beispielhaft und ohne auf diese Ausführungsform beschränkt zu sein - als Single-Needle-Blutbehandlungskassette ausgestaltete Kassette 1000 weist eine Single-Needle-Kammer 25 auf.

Die Kassette 1000 weist eine erfindungsgemäße Spülleitung 27 auf.

Die Spülleitung 27 ist im Beispiel der Fig. 1 zwischen der Zugabestelle 15 für Substituatflüssigkeit, bei welcher es sich um eine Fluidzuführeinrichtung handelt, und dem Prädilutionszugang 21 angeordnet.

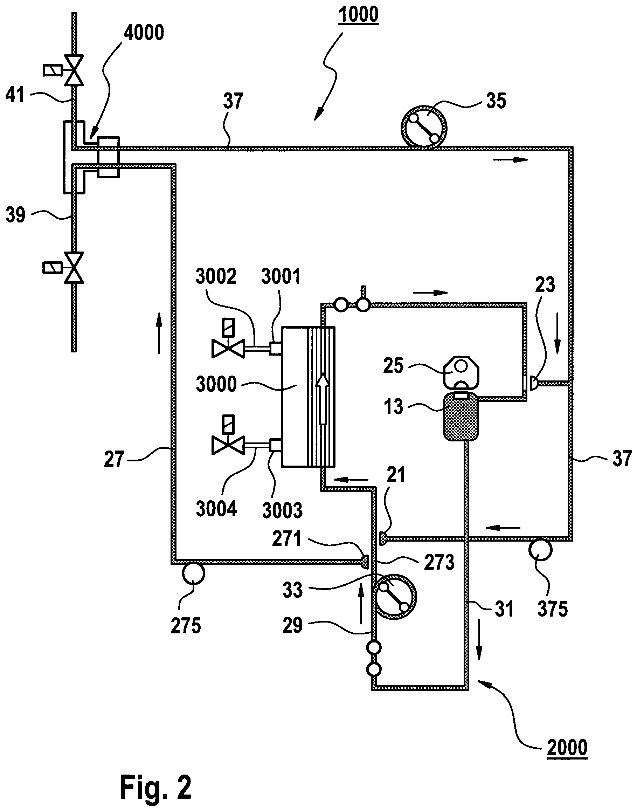

Im Folgenden wird mit Blick auf Fig. 2 die Durchführung des erfindungsgemäßen Verfahrens in einer rein beispielhaften Ausführungsform anhand einer Single-Needle-Behandlungskassette beschrieben.

Als Spül- und/oder Primingflüssigkeit wird beispielhaft Substituatflüssigkeit eingesetzt, wobei die vorliegende Erfindung wiederum nicht auf dieses Beispiel eingeschränkt ist.

**Fig. 2** zeigt schematisch vereinfacht eine erfindungsgemäße Funktionseinrichtung 1000 zum Durchführen eines erfindungsgemäßen Spül- und/oder Primingverfahrens.

Die Funktionseinrichtung 1000 weist einen extrakorporalen Blutkreislauf 2000 auf. Der extrakorporale Blutkreislauf 2000 weist einen arteriellen Leitungsabschnitt 29 sowie einen venösen Leitungsabschnitt 31 auf.

Der arterielle Leitungsabschnitt 29 und der venöse Leitungsabschnitt 31 sind kurzgeschlossen. Hierzu kann ein Standardgeradverbinder eingesetzt werden.

Im arteriellen Leitungsabschnitt 29 ist eine Blutpumpe 33 angeordnet.

Eine Substituatpumpe 35 ist in der Substituatleitung 37 angeordnet.

Eine Behandlungseinrichtung 3000 zwischen arteriellem Leitungsabschnitt 29 und venösem Leitungsabschnitt 31 dient der extrakorporalen Behandlung von Blut.

Beispielhaft als Dialysiereinrichtung (auch als Blutfilter bezeichnet) ausgestaltet, weist die Behandlungseinrichtung 3000 einen Dialysateinlass 3001 an einer zugehörigen Dialysatzuleitung 3002 sowie einen Dialysatauslass 3003 an einer zugehörigen Dialysatableitung 3004 auf.

Die Spülleitung 27 weist ein Spülventil 271 auf. Das Spülventil 271 kann allerdings auch Teil der Funktionseinrichtung 1000 sein, ferner auch Teil von Spülleitung 27 und Funktionseinrichtung 1000.

Die Spülleitung 27 geht von der Funktionseinrichtung 1000 bzw. einem Fluid führenden Abschnitt hiervon im Bereich einer Spülabzweigung 273 ab.

Die Spülleitung 27 weist ferner einen optionalen ersten Bluterkennungssensor 275 (z. B. einen optischen Sensor) auf.

Die Spülleitung 27 mündet in einen Substituat-Port 4000. Im Substituat-Port 4000 geht die Spülleitung 27 zu einer Drain-Leitung 39 ab.

Die Substituatleitung 37 führt vom Substituat-Port 4000 zum extrakorporalen Blutkreislauf 2000. Der Substituatleitung 37 wird frisches Substituat von einer Substituatflüssigkeitsquelle (nicht gezeigt) über eine Zuleitung 41 zugeführt.

In der Substituatleitung 37 ist ein zweiter Bluterkennungssensor 375 (z. B. ein optischer Sensor) angeordnet.

Zum Durchführen des Spül- und/oder Priming-Verfahrens gemäß der vorliegenden Erfindung wird zunächst die venöse Kammer 13 gefüllt.

Hierzu fördert die Substituatpumpe 35 zum Beispiel mit einem definierten Fluss - dieser kann vom Bedienpersonal eingestellt werden - Substituatflüssigkeit über den Postdilutionszugang 23 in die venöse Kammer 13. Eine venöse Klemme (in Fig. 2 nicht gezeigt) kann dabei geschlossen sein.

Die Substituatflüssigkeit gelangt von der venösen Kammer 13 durch ein geöffnetes Single-Needle-Ventil (in Fig. 2 nicht gezeigt), das mit der Single-Needle-Kammer 25 verbunden ist, in die Single-Needle-Kammer 25. Die Single-Needle-Kammer 25 ist vorzugsweise gegen die Umgebung geöffnet. Eine Entlüftung kann in bestimmten Ausführungsformen über einen zu öffnenden Anschluss an der Single-Needle-Kammer 25 - z. B. ein Entlüftungsventil an der Single-Needle-Kammer 25 - erfolgen.

Sobald ein Pegel in der venösen Kammer 13 erkannt wurde, werden - um den arteriellen und den venösen Leitungsabschnitt zu füllen - die arterielle und die venöse Klemme oder Schlauchklemme (in Fig. 2 jeweils nicht gezeigt) geöffnet.

Die Blutpumpe 33 wird mit definiertem Fluss gestartet, die Substituatpumpe 35 wird gestoppt.

Sobald der Pegel in der venösen Kammer 13 geeignet abgefallen ist, wird die Substituatpumpe 35 mit einem Volumenstrom, der größer als der Volumenstrom der Blutpumpe 33 ist, gestartet. Die Blutpumpe 33 läuft dazu weiter.

Dadurch wird die venöse Kammer 13 wieder aufgefüllt. Die Substituatpumpe 35 wird gestoppt, wenn der Pegel in der venösen Kammer 13 erkannt wurde und zusätzlich anschließend ein definiertes Volumen mittels der Substituatpumpe 35 gefördert wurde.

Wird am arteriellen Luftdetektor (in Fig. 2 nicht gezeigt) Flüssigkeit erkannt, fördert die Blutpumpe 33 ein definiertes Volumen zum Befüllen des Blutpumpenschlauchs und darüber hinaus solange weiter, bis der venöse Pegel abgefallen ist.

Zum Füllen der Behandlungseinrichtung 3000 werden die venöse Klemme und das Postdilutionsventil des Postdilutionszugangs 23 geschlossen.

Die Substituatpumpe 35 fördert über das geöffnete Prädilutionsventil des Prädilutionszugangs 21 solange Substituatflüssigkeit in den extrakorporalen Blutkreislauf, bis ein Pegel in der venösen Kammer 13 erkannt wird. Auf diese Weise wird die Behandlungseinrichtung 3000 gefüllt.

Anschließend wird das Spülen gestartet, wobei der extrakorporale Blutkreislauf 2000 vollständig mit Flüssigkeit gefüllt ist. In bestimmten Ausführungsformen der vorliegenden Erfindung kann das Spülen des extrakorporalen Kreislaufs 2000 unmittelbar nach dem Füllen erfolgen.

Das Spülen dient in bestimmten Ausführungsformen der Entfernung von Partikeln aus dem extrakorporalen Blutkreislauf 2000 und der Behandlungseinrichtung 3000, um deren Eindringen in den Patienten zu vermeiden.

Da der arterielle Leitungsabschnitt 29 und der venöse Leitungsabschnitt 31 kurzgeschlossen sind, ist ein Spülen im Singlepass nicht möglich. In solchen Ausführungsformen kann das erfindungsgemäße Verfahren vorteilhaft eine mögliche Rückzirkulation beachten und entsprechend vermeiden, wie nachfolgend beschrieben ist.

Die Behandlungseinrichtung 3000 ist blutseitig mit Substituatflüssigkeit gefüllt. Der extrakorporale Blutkreislauf 2000 ist luftfrei.

Um die Single-Needle-Kammer 25 zu füllen, fördert die Substituatpumpe 35 mit einem definierten Fluss über das Prädilutionsventil des Prädilutionszugangs 21.

Dabei wird Substituatflüssigkeit über die Behandlungseinrichtung 3000 in die Single-Needle-Kammer 25 eingebracht. Das Single-Needle-Ventil sowie ein Entlüftungsventil an der Single-Needle-Kammer 25 sind geöffnet.

Arterielle und venöse Klemmen (in Fig. 2 nicht gezeigt) sind geschlossen. Das Spülventil 271 ist geschlossen. Die Blutpumpe 33 wird nicht betrieben.

Sobald ein Pegel in der Single-Needle-Kammer 25 erkannt worden ist, wird diese volumetrisch gefüllt.

Wenn das volumetrische Füllen der Single-Needle-Kammer 25 abgeschlossen ist, wird das Entlüftungsventil an der Single-Needle-Kammer 25 geschlossen.

Das Spülventil 271 wird geöffnet.

Zudem, vorzugsweise gleichzeitig, werden die arterielle und die venöse Klemme geöffnet.

Die Blutpumpe 33 wird mit einem Fluss gestartet, der deutlich unter dem Fluss der Substituatpumpe 35 liegt. Dadurch teilt sich der Substituatstrom am Prädilutionszugang 21 auf. Ein Teil der Substituatflüssigkeit wird direkt in die Spülleitung 27 verworfen, der andere Teil - welcher zunächst nur durch die Förderrate der Blutpumpe 33 bestimmt ist - fließt durch die Behandlungseinrichtung 3000.

Der Strömungsweg der Substituatflüssigkeit ist in Fig. 2 durch einen Blockpfeil dargestellt.

Ist der Fluss der Substituatpumpe 35 ausreichend groß, dann gelangt die gesamte von der Blutpumpe 33 geförderte Substituatflüssigkeit in die Spülleitung 27. In bestimmten Ausführungsformen des erfindungsgemäßen Verfahrens kann eine Rückpulsation bei einem Verhältnis von Blutpumpenrate zu Substituatpumpenrate von 1:2 ausgeschlossen werden.

Auf diese Weise kann es vorteilhaft möglich sein, ein Überpulsen bzw. Überströmen der Substituatflüssigkeit über den Prädilutionszugang 21 zurück in die Substituatleitung 37 zu unterbinden. Eine Rückzirkulation der Substituatflüssigkeit kann so vorteilhaft vermieden werden.

Um den Pegel in der Single-Needle-Kammer 25 zu senken, wird mithilfe eines Single-Needle-Kompressors (in Fig. 2 nicht gezeigt) Luft in die Single-Needle-Kammer 25 gefördert.

Es kann so lange Luft gefördert werden, bis ein venöser Leveldetektor (in Fig. 2 nicht gezeigt) keinen Pegel mehr erkennt.

Um zu vermeiden, dass "verbrauchte", d. h. zum Spülen und/oder Primen der Funktionseinrichtung 1000 eingesetzte Flüssigkeit, wieder rückwärts bzw. entgegen der üblichen Strömungsrichtung durch die Behandlungseinrichtung 3000 gedrückt wird, wird der durch das Leeren der Single-Needle-Kammer 25 verursachte Volumenstrom in bestimmten Ausführungsformen so eingestellt, dass er den Fluss der Blutpumpe 33 nicht übersteigt.

Der venöse Leveldetektor und der Druck im Single-Needle-Tank werden benutzt, um den Flüssigkeitspegel in der venösen Kammer 13 so weit wie möglich zu senken.

Ist ein Sollpegel erreicht, wird die Blutpumpe 33 gestoppt.

Die venöse Klemme und das Entlüftungsventil an der Single-Needle-Kammer 25 werden geschlossen. Der Single-Needle-Kompressor wird angehalten.

Zur Bestimmung der Menge an Spülflüssigkeit kann der von der Blutpumpe 33 geförderte Fluss herangezogen werden. Ist ein definierter Wert erreicht, ist der Spülvorgang abgeschlossen.

Ist das nicht der Fall, kann in bestimmten Ausführungsformen erneut die Single-Needle-Kammer 25 gefüllt und das Spülen fortgesetzt werden.

**Bezugszeichenliste**

| **Bezugszeichen** | **Beschreibung** |
|---|---|
| 1000 | medizintechnische Funktionseinrichtung |
| 2000 | extrakorporaler Blutkreislauf |
| 3000 | Blutbehandlungseinrichtung |
| 3001 | Dialysateinlass |
| 3002 | Dialysatzuleitung |
| 3003 | Dialysatauslass |
| 3004 | Dialysatableitung |
| 4000 | Substituat-Port |
| 1 | arterieller Patientenanschluss |
| 3 | venöser Patientenanschluss |
| 5 | Konnektor für den Blutaustritt aus der Kassette 1000 |
| 7 | Konnektor für den Bluteintritt in die Kassette 1000 |
| 9 | arterielle Filterleitung |
| 11 | venöse Filterleitung |
| 13 | venöse Blutkammer |
| 15 | Zugabestelle für Substituatflüssigkeit |
| 17 | Konnektor für Substituataustritt aus der Kassette 1000 |
| 19 | Konnektor für Substituateintritt in die Kassette 1000 |
| 21 | Prädilutionszugang |
| 23 | Postdilutionszugang |
| 25 | Single-Needle-Kammer |
| 27 | Spülleitung |
| 271 | Spülventil |
| 273 | Spülabzweigung |
| 275 | erster Bluterkennungssensor |
| 29 | arterieller Leitungsabschnitt |
| 31 | venöser Leitungsabschnitt |
| 33 | Blutpumpe |
| 35 | Substituatpumpe |
| 37 | Substituatleitung |
| 375 | zweiter Bluterkennungssensor |
| 39 | Drain-Leitung |
| 41 | Zuleitung von Substituatflüssigkeit aus einer Substituatflüssigkeitsquelle |

## Patentansprüche

1. Spülleitung (27), zum Abführen eines Spülfluids aus einer mittels des Spülfluids gespülten medizintechnischen Funktionseinrichtung (1000) heraus,
- wobei die Spülleitung (27) einerseits verbunden ist oder zu ihrer Verbindung vorgesehen ist mit einem Abschnitt der medizintechnischen Funktionseinrichtung (1000);
**dadurch gekennzeichnet, dass**
die Spülleitung (27) andererseits verbunden ist mit einer Fluidzuführeinrichtung zum Einleiten eines medizinischen Fluids in die medizintechnische Funktionseinrichtung (1000) hinein, wobei die Fluidzuführeinrichtung ein zweilumiger Verbindungsabschnitt zum gleichzeitigen Führen von zwei Fluiden ist oder einen solchen aufweist.

2. Spülleitung (27) nach Anspruch 1, wobei die Fluidzuführeinrichtung (1000) ein Port zum Zuführen von Substituatflüssigkeit in die medizintechnische Funktionseinrichtung (1000) hinein ist.

3. Spülleitung (27) nach Anspruch 1 oder 2, wobei der zweilumige Verbindungsabschnitt ein erstes Lumen zur Aufnahme und/oder Leitung des medizinischen Fluids und ein zweites Lumen zur Aufnahme und/oder Leitung des Spülfluids aufweist.

4. Medizintechnische Funktionseinrichtung (1000), verbunden mit wenigstens einer Spülleitung (27) gemäß einem der Ansprüche 1 bis 3.

5. Medizintechnische Funktionseinrichtung (1000) nach Anspruch 4, ausgestaltet als extrakorporaler Blutschlauchsatz oder als Blutbehandlungskassette.

6. Medizintechnische Funktionseinrichtung (1000) nach Anspruch 5, ausgestaltet als Disposable-Blutbehandlungskassette.

7. Verfahren zum Spülen und/oder Primen einer medizintechnischen Funktionseinrichtung (1000) mit dem Schritt:
- Verwenden einer Spülleitung (27) gemäß einem der Ansprüche 1 bis 3 oder einer medizinischen Funktionseinrichtung (1000) gemäß einem der Ansprüche 4 bis 6.

8. Verfahren nach Anspruch 7 mit dem weiteren Schritt:
- wenigstens teilweises Austragen bzw. Ableiten des Spülfluids aus der gespülten medizintechnischen Funktionseinrichtung (1000) durch die Spülleitung (27) in einen Port oder Maschinenanschluss zum Einbringen von Substituatflüssigkeit aus einer medizintechnischen Behandlungsvorrichtung in die medizintechnische Funktionseinrichtung (1000).

## Claims

1. A rinsing line (27) for discharging a rinsing fluid from a medical-technical functional device (1000) rinsed by means of the rinsing fluid,
- wherein, on the one hand, the rinsing line (27) is connected or provided for its connection with a portion of the medical-technical functional device (1000);
**characterized in that**
on the other hand, the rinsing line (27) is connected with a fluid supply device for introducing a medical fluid into the medical-technical functional device (1000), the fluid supply device being or comprising a two-lumen connecting portion for simultaneously guiding two fluids.

2. The rinsing line (27) according to claim 1, wherein the fluid supply device is a port for supplying substitute liquid into the medical-technical functional device (1000).

3. The rinsing line (27) according to claim 1 or 2, wherein the two-lumen connecting portion comprises a first lumen for receiving and/or directing the medical fluid and a second lumen for receiving and/or directing the rinsing fluid.

4. A medical-technical functional device (1000) connected with at least one rinsing line (27) according to anyone of claims 1 to 3.

5. The medical-technical functional device (1000) according to claim 4, designed as an extracorporeal blood tubing set or as a blood treatment cassette.

6. The medical-technical functional device (1000) according to claim 5, designed as a disposable blood treatment cassette.

7. A method of rinsing and/or priming a medical-technical functional device (1000) comprising the step of:
- using a rinsing line (27) according to anyone of claims 1 to 3 or a medical-technical functional device (1000) according to anyone of claims 4 to 6.

8. The method according to claim 7 comprising the further step of:
- diverting or discharging, at least partially, the rinsing fluid from the rinsed medical-technical functional device (1000) through the rinsing line (27) into a port or a machine connection in order to introduce substitute liquid from a medical-technical treatment apparatus into the medical-technical device (1000).

## Revendications

1. Une conduite de rinçage (27) permettant l'évacuation d'un fluide de rinçage d'un dispositif de fonctionnement médico-technique (1000) rincé au moyen du fluide de rinçage,
- où, d'une part, la conduite de rinçage (27) est reliée ou prévue pour être reliée à une section du dispositif de fonctionnement médico-technique (1000);
**caractérisée en ce que**
d'autre part, la conduite de rinçage (27) est reliée à un dispositif d'alimentation en fluide afin d'introduire un fluide médical dans le dispositif de fonctionnement médico-technique (1000), le dispositif d'alimentation en fluide étant ou comprenant une section de liaison à deux lumières pour le guidage simultané de deux fluides.

2. La conduite de rinçage (27) selon la première revendication, où le dispositif d'alimentation en fluide est un port permettant de fournir du liquide de substitut dans le dispositif de fonctionnement médico-technique (1000).

3. La conduite de rinçage (27) selon la revendication 1 ou 2, où la section de liaison à deux lumières comprend une première lumière pour recevoir et/ou diriger le fluide médical ainsi qu'une seconde lumière pour recevoir et/ou diriger le fluide de rinçage.

4. Un dispositif de fonctionnement médico-technique (1000) relié au moins à une conduite de rinçage (27) selon l'une quelconque des revendications 1 à 3.

5. Le dispositif de fonctionnement médico-technique (1000), selon la revendication 4, conçu sous la forme d'un assortiment de tuyaux sanguins extracorporels ou d'une cassette de traitement du sang.

6. Le dispositif de fonctionnement médico-technique (1000), selon la revendication 5, conçu sous la forme d'une cassette de traitement du sang à usage unique.

7. Un procédé de rinçage et/ou d'amorçage d'un dispositif de fonctionnement médico-technique (1000) comprenant l'étape consistant à:
- utiliser une conduite de rinçage (27) selon l'une quelconque des revendications 1 à 3 ou un dispositif de fonctionnement médico-technique (1000) selon l'une quelconque des revendications 4 à 6.

8. Le procédé selon la revendication 7 comprenant en outre l'étape consistant à:
- dériver voire évacuer, au moins partiellement, le fluide de rinçage du dispositif de fonctionnement médico-technique (1000) rincé au travers de la conduite de rinçage (27) dans un port ou un branchement de machine afin d'introduire du liquide de substitut provenant d'un appareil de traitement médico-technique dans le dispositif de fonctionnement médico-technique (1000).
